# EUROPEAN PATENT APPLICATION

(11) **EP 4 464 696 A1**
(43) Date of publication of application: **20.11.2024**
(21) Application number: 23740464.5
(22) Date of filing: 12.01.2023
(51) Int. Cl.: C07D 301/12, C08G 59/22, C08G 59/50, C08G 59/44, C08G 59/40, C08G 59/68

(54) **CHEMICALLY DEGRADABLE EPOXY COMPOUND, METHOD FOR PREPARING SAME, EPOXY COMPOSITE MATERIAL COMPRISING SAME, AND DECOMPOSITION METHOD THEREOF**

(30) Priority: 12.01.2022 KR 20220004750
(71) Applicant: Konkuk University Industrial Cooperation Corp., Gwangjin-gu Seoul 05029 (KR)
(72) Inventor: GOH, Mun Ju, Seoul 05029 (KR); HONG, Young Gi, Seoul 05016 (KR); JEONG, Ji Su, Gwangju 61126 (KR)
(74) Representative: EP&C
(86) International application number: PCT/KR2023/000557
(87) International publication number: WO 2023/136620

(57) **Abstract**

The present disclosure relates to a recyclable epoxy compound containing an α,β-unsaturated ketone group and/or a hydroxy ketone group bonded through an aldol reaction between a ketone group and an aldehyde group containing a hydroxyl group among non-toxic natural materials, without using a bisphenol A type epoxy, a toxic material. In addition, the present disclosure relates to a method of preparing the same compound, an epoxy composite material containing the same compound, and a method of degrading the same composite material.

## Description

### Technical Field

The present disclosure relates to a chemically degradable epoxy compound, a method of preparing the same compound, an epoxy composite material containing the same compound, and a method of degrading the same composite material. More specifically, the present disclosure relates to a recyclable epoxy compound containing an α,β-unsaturated ketone group and/or a hydroxy ketone group bonded through an aldol reaction between a ketone group and an aldehyde group containing a hydroxyl group among non-toxic natural materials, without using a bisphenol A type epoxy, a toxic material, a method of preparing the same compound, an epoxy composite material containing the same compound, and a method of degrading the same composite material.

### Background Art

Epoxy is a thermosetting resin formed by mixing an epoxy monomer having two or more epoxy groups in a molecule with a curing agent such as diamine, triamine, or imidazole, and composed of a network polymer formed by ring-opening of an epoxy group. Among these epoxies, polymer epoxy resins made from bisphenol A (BPA), a raw material, are used worldwide as polymer materials worth 15 trillion won, including paint adhesives, electrical and electronic materials, and composite materials.

However, bisphenol A, the raw material, is designated as a regulated substance worldwide, including in Europe and the United States, due to the hazards of the raw material to the human body. Since the development of alternative materials is becoming an issue, a variety of BPA-free epoxies are currently being developed. However, due to difficulties in physical strength and process application, commercialization and utilization have yet to be achieved overseas and domestically.

In addition, existing BPA epoxy resins are insoluble in solvents and unmeltable by heat, thus having the disadvantage of not being recyclable. Furthermore, environmental pollution problems resulting from applied products to which discarded epoxy resins are applied are emerging as serious social issues. As a result, developing a recyclable method and an alternative epoxy resin capable of being chemically degradable is urgently required as one way to reduce costs and solve such environmental pollution problems.

### Disclosure

### Technical Problem

The present disclosure has been made to solve the problems of the related art as described above. An objective of the present disclosure is to provide an epoxy compound containing a novel natural product-derived α,β-unsaturated ketone endowed with recyclability.

Another objective of the present disclosure is to provide a method of preparing the epoxy compound containing the novel natural product-derived α,β-unsaturated ketone endowed with recyclability.

A further objective of the present disclosure is to provide an epoxy composite material containing the epoxy compound described above.

A yet further objective of the present disclosure is to provide a method of degrading the epoxy composite material.

A still yet further objective of the present disclosure is to provide a wind blade including the epoxy compound.

### Technical Solution

In order to accomplish the above objectives, the present disclosure provides a recyclable epoxy compound represented by Formula 3 or 4 and being chemically degradable.

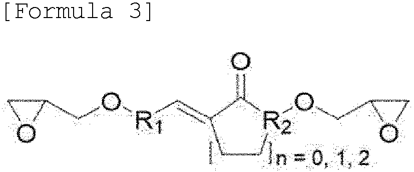

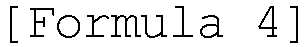

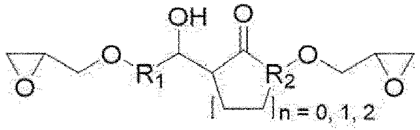

In Formulas 3 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 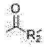
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

In one embodiment of the present disclosure, the compound represented by Formula 3 may be a compound represented by Formula 7.

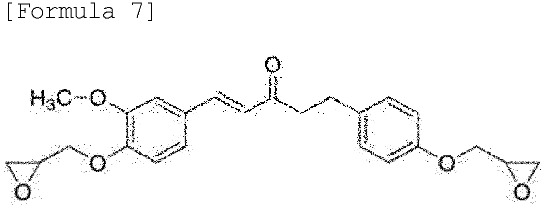

In one embodiment of the present disclosure, the compound represented by Formula 4 may be a compound represented by Formula 8.

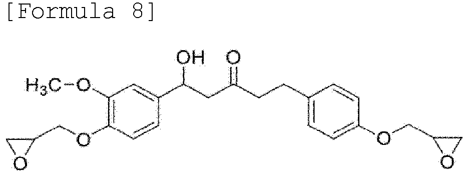

In order to accomplish the above objectives, the present disclosure provides a method of preparing a recyclable epoxy compound represented by Formula 3 and being chemically degradable, the method including: (a) causing an aldol reaction between an aldehyde group-containing compound and a ketone group-containing compound in the presence of a solvent and a catalyst to prepare a compound represented by Formula 1; (b) performing heat treatment on the compound represented by Formula 1 to prepare a compound represented by Formula 2; and (c) causing a reaction between the compound represented by Formula 2 and epichlorohydrin in the presence of a base.

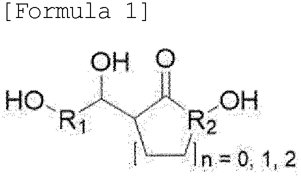

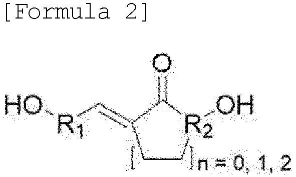

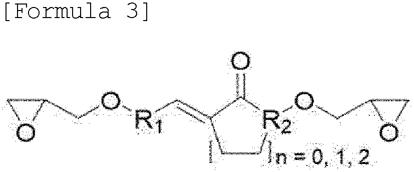

In Formulas 1 to 3,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 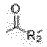
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

In one embodiment of the present disclosure, the aldehyde-group containing compound may be selected from the group consisting of 4-hydroxy-3-methoxybenzaldehyde, 4-hydroxybenzaldehyde, 3,4-dihydroxybenzaldehyde, p-hydroxycinnamaldehyde, 1-(hydroxymethyl)-cyclohexanecarboxaldehyde, 1-(hydroxymethyl)-4-isopropylcyclohexanecarbaldehyde, 3-hydroxycyclopentane-1-carbaldehyde, 4-hydroxycyclohexanecarboxaldehyde, 4-hydroxy-3-(2-propen-1-yl)cyclohexanecarboxaldehyde, 4-(hydroxymethyl)benzaldehyde, 2-(hydroxymethyl)benzaldehyde, 4-(2-hydroxyethyl)benzaldehyde, 5,6,9,10-tetrahydroxyphenanthro[9,10-b]furan-2-carboxaldehyde, 4-(3-hydroxy-4-oxo-4H-1-benzopyran-2-yl)benzaldehyde, trans-p-coumaraldehyde, (Z)-3-(4-hydroxy-3-methoxyphenyl)prop-2-enal, (E)-3-(4-hydroxy-3-methoxyphenyl)prop-2-enal, trans-4-(2,5dihydroxyphenyl)cyclohexanecarboxaldehyde, 4-hydroxy-7-benzofurancarboxaldehyde, 3-hydroxybutanal, 2,2-dimethyl-3hydroxypropane, glycolaldehyde, 3-hydroxypropionaldehyde, glyceraldehyde, 6-hydroxyhexanal, and hydroxypivalaldehyde.

In one embodiment of the present disclosure, the ketone group-containing compound may be selected from the group consisting of 4-(4-hydroxyphenyl)-2-butanone, 4'-hydroxyacetophenone, 4'-hydroxy-3'-methylacetophenone, 1-(6-hydroxy[1,1'-biphenyl]-3-yl)ethanone, 1-(4-hydroxycyclohexyl)ethanone, 4-hydroxybenzylacetone, 4-hydroxyphenylacetone, 2'-hydroxyacetophenone, 1-[4-[(1E)-2-hydroxyethenyl]phenyl]ethanone, 2,4-diacetyl-5-hydroxy-5-methyl-3-phenylcyclohexanone, (3E)-6-(4-hydroxyphenyl)-3-hexen-2-one, 2,4-diacetyl-5-hydroxy-3-(4-hydroxy-3-methoxyphenyl)-5-methylcyclohexanone, (3E)-4-(4-hydroxy-3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(4-hydroxyphenyl)-1-(3-methyl-1,2-benzisoxazol-5-yl)-2-propen-1-one, hydroxyacetone, 3-hydroxy-2-propanone, hydroxy-2-pentanone, 6-hydroxy-2-hexanone, 10-hydroxy-2-decanone, 8-hydroxy-2-octanone, 7-hydroxy-2-heptanone, (3S,4R)-3,4-dihydroxy-2,8-nonanedione, and 4-hydroxy-4-methyl-2,8-nonanedione.

In one embodiment of the present disclosure, the solvent may be selected from the group consisting of dichloromethane, tetrahydrofuran, hexamethylphosphoramide, hexane, toluene, dimethyl sulfoxide, ethanol, and water.

In one embodiment of the present disclosure, the catalyst may be selected from the group consisting of titanium tetrachloride, proline, N-ethylpiperidine, magnesium iodide, samarium trichloride, butyllithium, 1-piperazineethaneamine, diethylzinc, bis[(4R)-4-hydroxy-L-prolinato-κN1,κO2]copper, tin (II) triflate, boron trifluoride etherate, and tetraethylammonium p-toluenesulfonate.

In one embodiment of the present disclosure, the solvent may be water, and the catalyst may be proline.

In one embodiment of the present disclosure, the heat treatment may be performed at a temperature in a range of 60°C to 90°C.

In one embodiment of the present disclosure, the base may be selected from the group consisting of sodium hydroxide, potassium carbonate, 4-methyl-2-phenyl-1H-imidazole-5-methanol, and hexadecyltrimethylammonium bromide.

In one embodiment of the present disclosure, the reaction in the (c) may be performed at a temperature in a range of 10°C to 50°C.

In order to accomplish the above objectives, the present disclosure provides a method of preparing a recyclable epoxy compound represented by Formula 4 and being chemically degradable, the method including: (d) causing an aldol reaction between an aldehyde group-containing compound and a ketone group-containing compound in the presence of a solvent and a catalyst to prepare a compound represented by Formula 1; and (e) causing a reaction between the compound represented by Formula 1 and epichlorohydrin in the presence of a base.

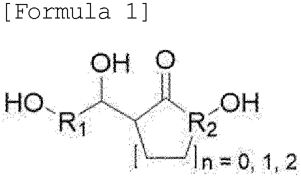

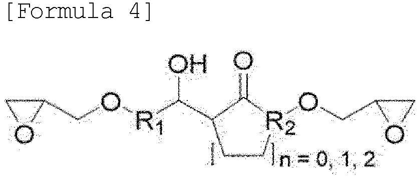

In Formulas 1 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 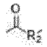
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

In one embodiment of the present disclosure, the aldehyde group-containing compound may be selected from the group consisting of 4-hydroxy-3-methoxybenzaldehyde, 4-hydroxybenzaldehyde, 3,4-dihydroxybenzaldehyde, p-hydroxycinnamaldehyde, 1-(hydroxymethyl)-cyclohexanecarboxaldehyde, 1-(hydroxymethyl)-4-isopropylcyclohexanecarbaldehyde, 3-hydroxycyclopentane-1-carbaldehyde, 4-hydroxycyclohexanecarboxaldehyde, 4-hydroxy-3-(2-propen-1-yl)cyclohexanecarboxaldehyde, 4-(hydroxymethyl)benzaldehyde, 2-(hydroxymethyl)benzaldehyde, 4-(2-hydroxyethyl)benzaldehyde, 5,6,9,10-tetrahydroxyphenanthro[9,10-b]furan-2-carboxaldehyde, 4-(3-hydroxy-4-oxo-4H-1-benzopyran-2-yl)benzaldehyde, trans-p-coumaraldehyde, (Z)-3-(4-hydroxy-3-methoxyphenyl)prop-2-enal, (E)-3-(4-hydroxy-3-methoxyphenyl)prop-2-enal, trans-4-(2,5dihydroxyphenyl)cyclohexanecarboxaldehyde, 4-hydroxy-7-benzofurancarboxaldehyde, 3-hydroxybutanal, 2,2-dimethyl-3hydroxypropane, glycolaldehyde, 3-hydroxypropionaldehyde, glyceraldehyde, 6-hydroxyhexanal, and hydroxypivalaldehyde.

In one embodiment of the present disclosure, the ketone group-containing compound may be selected from the group consisting of 4-(4-hydroxyphenyl)-2-butanone, 4'-hydroxyacetophenone, 4'-hydroxy-3'-methylacetophenone, 1-(6-hydroxy[1,1'-biphenyl]-3-yl)ethanone, 1-(4-hydroxycyclohexyl)ethanone, 4-hydroxybenzylacetone, 4-hydroxyphenylacetone, 2'-hydroxyacetophenone, 1-[4-[(1E)-2-hydroxyethenyl]phenyl]ethanone, 2,4-diacetyl-5-hydroxy-5-methyl-3-phenylcyclohexanone, (3E)-6-(4-hydroxyphenyl)-3-hexen-2-one, 2,4-diacetyl-5-hydroxy-3-(4-hydroxy-3-methoxyphenyl)-5-methylcyclohexanone, (3E)-4-(4-hydroxy-3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(4-hydroxyphenyl)-1-(3-methyl-1,2-benzisoxazol-5-yl)-2-propen-1-one, hydroxyacetone, 3-hydroxy-2-propanone, hydroxy-2-pentanone, 6-hydroxy-2-hexanone, 10-hydroxy-2-decanone, 8-hydroxy-2-octanone, 7-hydroxy-2-heptanone, (3S,4R)-3,4-dihydroxy-2,8-nonanedione, and 4-hydroxy-4-methyl-2,8-nonanedione.

In one embodiment of the present disclosure, the solvent may be selected from the group consisting of dichloromethane, tetrahydrofuran, hexamethylphosphoramide, hexane, toluene, dimethyl sulfoxide, ethanol, and water.

In one embodiment of the present disclosure, the catalyst may be selected from the group consisting of titanium tetrachloride, proline, N-ethylpiperidine, magnesium iodide, samarium trichloride, butyllithium, 1-piperazineethaneamine, diethylzinc, bis[(4R)-4-hydroxy-L-prolinato-κN1,κO2]copper, tin (II) triflate, boron trifluoride etherate, and tetraethylammonium p-toluenesulfonate.

In one embodiment of the present disclosure, the solvent may be water, and the catalyst may be proline.

In one embodiment of the present disclosure, the base may be selected from the group consisting of sodium hydroxide, potassium carbonate, 4-methyl-2-phenyl-1H-imidazole-5-methanol, and hexadecyltrimethylammonium bromide.

In one embodiment of the present disclosure, the reaction in the (e) may be performed at a temperature in a range of 10°C to 50°C.

In order to accomplish the above objectives, the present disclosure provides an epoxy composite material containing: at least one epoxy compound selected from the group consisting of the epoxy compounds represented by Formulas 3 and 4; and a curing agent.

In one embodiment of the present disclosure, the curing agent may be selected from the group consisting of 2,6-dimethylaniline, hexamethylenediamine, isophorone diamine, diaminodiphenyl sulfone, 1,3benzenedimethaneanine, and dicyandiamide.

In order to accomplish the above objectives, the present disclosure provides a method of degrading an epoxy composite material, the method including causing a reaction of the epoxy composite material in the presence of a catalyst.

In one embodiment of the present disclosure, the catalyst may be selected from the group consisting of titanium tetrachloride, ammonium chloride, trifluoroacetic acid, hydroxyamine hydrochloride, potassium hydroxide, sodium isopropoxide, tetrabutylammonium hydroxide, ethylene glycol, pyrrolidine, sodium periodate, cesium carbonate, alumina, and silver.

In one embodiment of the present disclosure, the reaction may be performed at a temperature in a range of 0°C to 40°C.

In order to accomplish the above objectives, the present disclosure provides a wind blade including the epoxy compound as a raw material.

### Advantageous Effects

The present disclosure has the advantage of providing: a recyclable epoxy containing an α,β-unsaturated ketone group or hydroxy ketone group bonded through an aldol reaction between a ketone group and an aldehyde group containing a hydroxyl group among non-toxic natural materials, without using a bisphenol A type epoxy, a toxic material; a method of preparing the same epoxy; and a method of improving thermal properties, physical properties, and recyclability of an epoxy composite material by using the epoxy.

### Description of Drawings

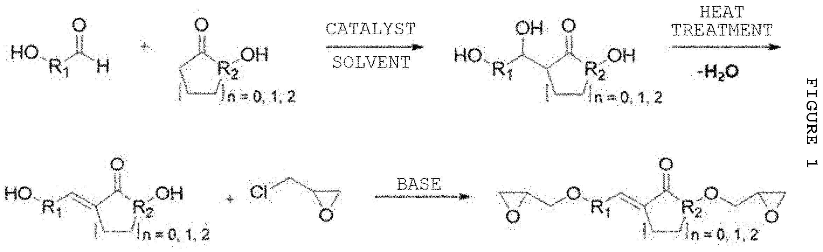

FIG. 1 is a diagram illustrating a reaction formula for a process of preparing a recyclable epoxy compound containing an α,β-unsaturated ketone according to one embodiment of the present disclosure;
FIG. 2 is a diagram showing ¹H-NMR analysis results of an α,β-unsaturated ketone epoxy compound according to one embodiment of the present disclosure;
FIG. 3 is a diagram showing IR spectra of an α,β-unsaturated ketone epoxy compound according to one embodiment of the present disclosure;
FIG. 4 is a diagram illustrating a reaction formula for a process of preparing an α,β-unsaturated ketone epoxy compound according to one embodiment of the present disclosure;
FIG. 5 is a diagram showing results of confirming the recyclability of an epoxy composite material containing a recyclable epoxy compound containing an α,β-unsaturated ketone according to one embodiment of the present disclosure;
FIG. 6 is a diagram showing a graph of a melting transition temperature determined from differential scanning calorimetry (DSC) results to examine the workability of a recyclable epoxy compound containing an α,β-unsaturated ketone according to one embodiment of the present disclosure;
FIG. 7 is a diagram showing a graph of a curing temperature determined from differential scanning calorimetry (DSC) results to examine the workability of a recyclable epoxy compound containing an α,β-unsaturated ketone according to one embodiment of the present disclosure;
FIG. 8 is a diagram showing a graph of a glass transition temperature determined from differential scanning calorimetry (DSC) results to examine the workability of a recyclable epoxy compound containing an α,β-unsaturated ketone according to one embodiment of the present disclosure; and
FIG. 9 is a diagram illustrating results of confirming a recycling process of an epoxy composite material containing a recyclable epoxy compound containing an α,β-unsaturated ketone according to one embodiment of the present disclosure.

### Best Mode

Hereinafter, the present disclosure will be described in detail with embodiments. Although the present disclosure has been described with reference to the embodiments shown in the drawings, the embodiments are described as one example, whereby the technical spirit, key configuration, and operation of the present disclosure are not limited.

A first embodiment of the present disclosure provides a recyclable epoxy compound represented by Formula 3 or 4 and being chemically degradable.

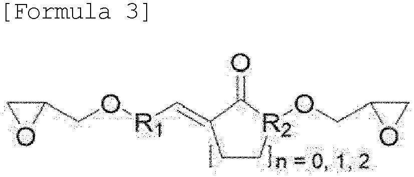

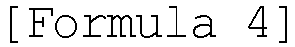

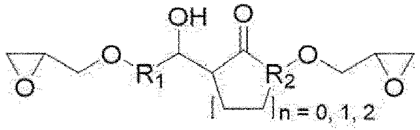

In Formulas 3 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, 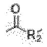
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

The epoxy compound has a melting point in a range of 130°C to 150°C, preferably about 140°C, and has properties of being capable of cross-linking with a curing agent as well as being chemically degradable and recyclable. As a result, the use of the epoxy compound has the advantage that a nanocomposite material with improved thermal and physical properties can be prepared.

Examples of the epoxy compound may include Formula 7 or 8 below, but are not limited thereto.

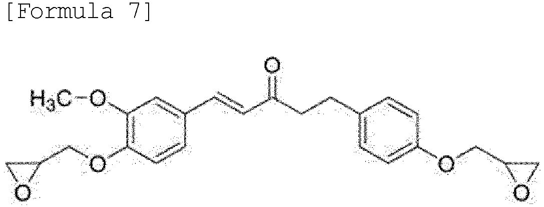

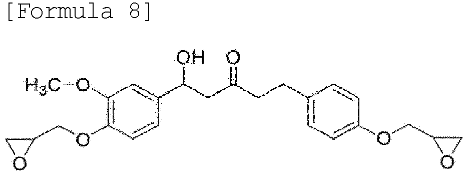

A second embodiment of the present disclosure provides a method of preparing a recyclable epoxy compound represented by Formula 3 and being chemically degradable, the method including:
(a) causing an aldol reaction between an aldehyde group-containing compound and a ketone group-containing compound in the presence of a solvent and a catalyst to prepare a compound represented by Formula 1 (Reaction Formula 1); (b) performing heat treatment on the compound represented by Formula 1 to prepare a compound represented by Formula 2 (Reaction Formula 2); and (c) causing a reaction between the compound represented by Formula 2 and epichlorohydrin in the presence of a base (Reaction Formula 3).

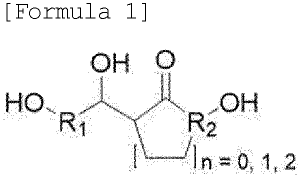

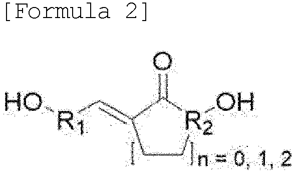

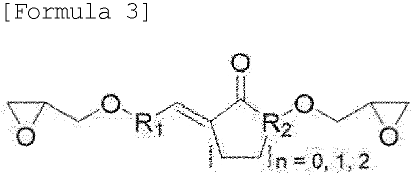

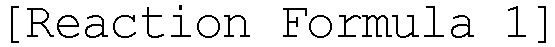

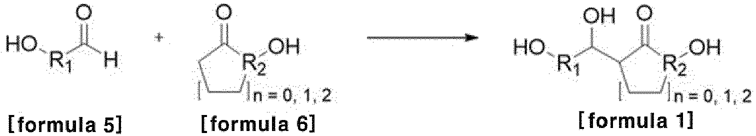

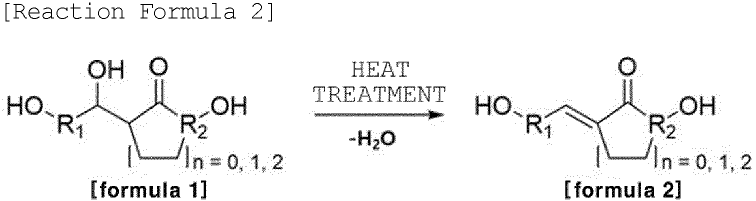

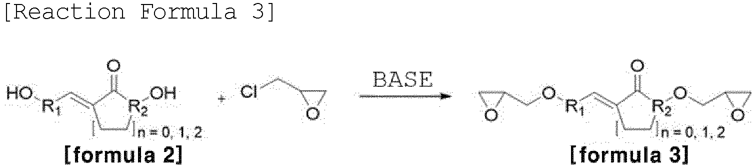

In Formulas 1 to 3, R₁ may be selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate. However, R₁ is not limited thereto. 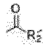 may be selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione. However, 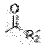 is not limited thereto.

A third embodiment of the present disclosure provides a method of preparing a recyclable epoxy compound represented by Formula 4 and being chemically degradable, the method including: (d) causing an aldol reaction between an aldehyde group-containing compound and a ketone group-containing compound in the presence of a solvent and a catalyst to prepare a compound represented by Formula 1 (Reaction Formula 1); and (e) causing a reaction between the compound represented by Formula 1 and epichlorohydrin in the presence of a base (Reaction Formula 4).

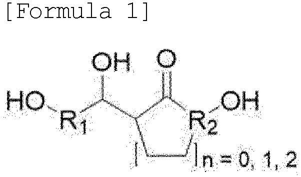

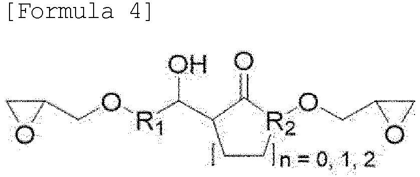

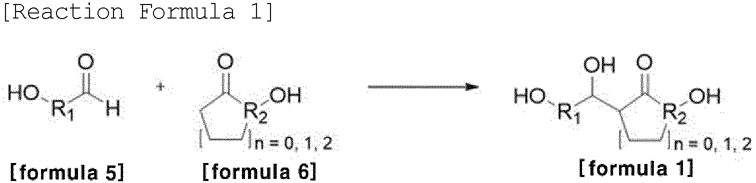

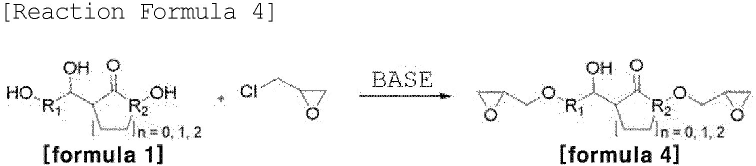

In Formulas 1 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 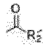
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

In Reaction Formula 1, the aldehyde group-containing compound of Formula 5 may be selected from the group consisting of 4-hydroxy-3-methoxybenzaldehyde, 4-hydroxybenzaldehyde, 3,4-dihydroxybenzaldehyde, p-hydroxycinnamaldehyde, 1-(hydroxymethyl)-cyclohexanecarboxaldehyde, 1-(hydroxymethyl)-4-isopropylcyclohexanecarbaldehyde, 3-hydroxycyclopentane-1-carbaldehyde, 4-hydroxycyclohexanecarboxaldehyde, 4-hydroxy-3-(2-propen-1-yl)cyclohexanecarboxaldehyde, 4-(hydroxymethyl)benzaldehyde, 2-(hydroxymethyl)benzaldehyde, 4-(2-hydroxyethyl)benzaldehyde, 5,6,9,10-tetrahydroxyphenanthro[9,10-b]furan-2-carboxaldehyde, 4-(3-hydroxy-4-oxo-4H-1-benzopyran-2-yl)benzaldehyde, trans-p-coumaraldehyde, (Z)-3-(4-hydroxy-3-methoxyphenyl)prop-2-enal, (E)-3-(4-hydroxy-3-methoxyphenyl)prop-2-enal, trans-4-(2,5dihydroxyphenyl)cyclohexanecarboxaldehyde, 4-hydroxy-7-benzofurancarboxaldehyde, 3-hydroxybutanal, 2,2-dimethyl-3hydroxypropane, glycolaldehyde, 3-hydroxypropionaldehyde, glyceraldehyde, 6-hydroxyhexanal, and hydroxypivalaldehyde. However, the aldehyde group-containing compound of Formula 5 is not limited thereto.

In Reaction Formula 1, the ketone group-containing compound of Formula 6 may be selected from the group consisting of 4-(4-hydroxyphenyl)-2-butanone, 4'-hydroxyacetophenone, 4'-hydroxy-3'-methylacetophenone, 1-(6-hydroxy[1,1'-biphenyl]-3-yl)ethanone, 1-(4-hydroxycyclohexyl)ethanone, 4-hydroxybenzylacetone, 4-hydroxyphenylacetone, 2'-hydroxyacetophenone, 1-[4-[(1E)-2-hydroxyethenyl]phenyl]ethanone, 2,4-diacetyl-5-hydroxy-5-methyl-3-phenylcyclohexanone, (3E)-6-(4-hydroxyphenyl)-3-hexen-2-one, 2,4-diacetyl-5-hydroxy-3-(4-hydroxy-3-methoxyphenyl)-5-methylcyclohexanone, (3E)-4-(4-hydroxy-3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(4-hydroxyphenyl)-1-(3-methyl-1,2-benzisoxazol-5-yl)-2-propen-1-one, hydroxyacetone, 3-hydroxy-2-propanone, hydroxy-2-pentanone, 6-hydroxy-2-hexanone, 10-hydroxy-2-decanone, 8-hydroxy-2-octanone, 7-hydroxy-2-heptanone, (3S,4R)-3,4-dihydroxy-2,8-nonanedione, and 4-hydroxy-4-methyl-2,8-nonanedione. However, the ketone group-containing compound of Formula 6 is not limited thereto.

In Reaction Formula 1, the solvent may be selected from the group consisting of dichloromethane, tetrahydrofuran, hexamethylphosphoramide, hexane, toluene, dimethyl sulfoxide, ethanol, and water. However, the solvent is not limited thereto.

In Reaction Formula 1, the catalyst may be selected from the group consisting of titanium tetrachloride, proline, N-ethylpiperidine, magnesium iodide, samarium trichloride, butyllithium, 1-piperazineethaneamine, diethylzinc, bis[(4R)-4-hydroxy-L-prolinato-κN1,κO2]copper, tin (II) triflate, boron trifluoride etherate, and tetraethylammonium p-toluenesulfonate. However, the catalyst is not limited thereto.

In Reaction Formula 1, the solvent may be water, and the catalyst may be proline. However, the solvent and the catalyst are not limited thereto. As described above, in the present disclosure, proline, an organic catalyst, is used instead of (heavy) metal catalysts used in the synthesis of existing epoxy resins. In addition, water is used as the solvent instead of existing petrochemical-derived solvents. Thus, there is an advantage that epoxy resins can be prepared in an environmentally friendly way.

In Reaction Formula 1, after completion of the reaction, an aqueous solution of ammonium chloride and the like may be used to stop the reaction, and the compound represented by Formula 1 may then be obtained. However, the present disclosure is not limited thereto.

In Reaction Formula 2, the heat treatment may be performed at a temperature in a range of 60°C to 90°C, preferably in the range of 70°C to 80°C, for 1 hour to 4 hours, preferably 2 hours. However, the present disclosure is not limited thereto. As water is generated from the hydroxyl group in Formula 1 by the heat treatment, the compound represented by Formula 2 and containing an unsaturated ketone group can be prepared.

In Reaction Formulas 3 and 4, the base may be selected from the group consisting of sodium hydroxide, potassium carbonate, 4-methyl-2-phenyl-1H-imidazole-5-methanol, and hexadecyltrimethylammonium bromide. However, the base is not limited thereto.

In Reaction Formulas 3 and 4, the reaction may be performed at a temperature in a range of 10°C to 50°C, and preferably in the range of 20°C to 40°C. However, the present disclosure is not limited thereto.

A fourth embodiment of the present disclosure provides an epoxy composite material containing: at least one epoxy compound selected from the group consisting of the epoxy compounds represented by Formulas 3 and 4; and a curing agent.

The curing agent may be selected from the group consisting of 2,6-dimethylaniline, hexamethylenediamine, isophorone diamine, diaminodiphenyl sulfone, 1,3benzenedimethaneanine, and dicyandiamide. However, the curing agent is not limited thereto.

The epoxy composite material may be, for example, prepared by first mixing the epoxy compound represented by Formula 3 and/or Formula 4 with the curing agent, pouring the mixture into a mold, and then performing heat-pressure curing at a temperature in a range of 150°C to 250°C, preferably in the range of 175°C to 220°C, for 1 hour to 5 hours, preferably 1 hour to 3 hours. However, the present disclosure is not limited thereto.

The curing agent in the epoxy composite material may be used in an amount in a range of 0.5 wt% to 50 wt%, preferably in the range of 1 wt% to 40 wt%, and more preferably in the range of 5 wt% to 20 wt%. However, the amount of the curing agent is not limited thereto.

The epoxy composite material may further include a filler. Specific examples of the filler may include quartz, silica, silicon oxide, fumed silica, fumed quartz, natural silica, synthetic silica, natural aluminum oxide, synthetic aluminum oxide, aluminum trihydroxide, aluminum-oxide-hydroxide, magnesium hydroxide, aluminum hydroxide oxide, boron nitride, aluminum nitride, silicon nitride, silicon carbide, mica, calcium carbonate, lithium aluminum silicate, zinc oxide, aluminum nitride, mullite, wollastonite, talcum, kaolin, bentonite, boehmite, exonolite, andalusite, zeolite, dolomite, vermiculite, muscovite, nepheline, albite, microline, slate, aluminum powder, silver, graphite, synthetic graphite, natural graphite, amorphous graphite, flake graphite, vein graphite, expandable/intumescent graphite, antimony oxide, borate, molybdate, stannate (including zinc stannate), phosphinate, ammonium polyphosphate, melamine polyphosphate, melamine salt, zinc sulfide, red phosphorus, layered clay, gold, carbon, single-walled or multi-walled carbon nanotube, graphene, glass powder, glass fiber, glass fabric, glass sheet, carbon fiber, other organic or inorganic particulate fillers, or mixtures thereof. However, the filler is not limited thereto.

A fifth embodiment of the present disclosure provides a method of degrading the epoxy composite material, the method including causing a reaction of the epoxy composite material in the presence of a catalyst.

The catalyst may be selected from the group consisting of titanium tetrachloride, ammonium chloride, trifluoroacetic acid, hydroxyamine hydrochloride, potassium hydroxide, sodium isopropoxide, tetrabutylammonium hydroxide, ethylene glycol, pyrrolidine, sodium periodate, cesium carbonate, alumina, and silver. However, the catalyst is not limited thereto.

The reaction may be performed at a temperature in a range of 0°C to 40°C, preferably in the range of 10°C to 30°C, but is not limited thereto.

Reaction Formula 5 illustrates a mechanism by which the epoxy composite material is degraded.

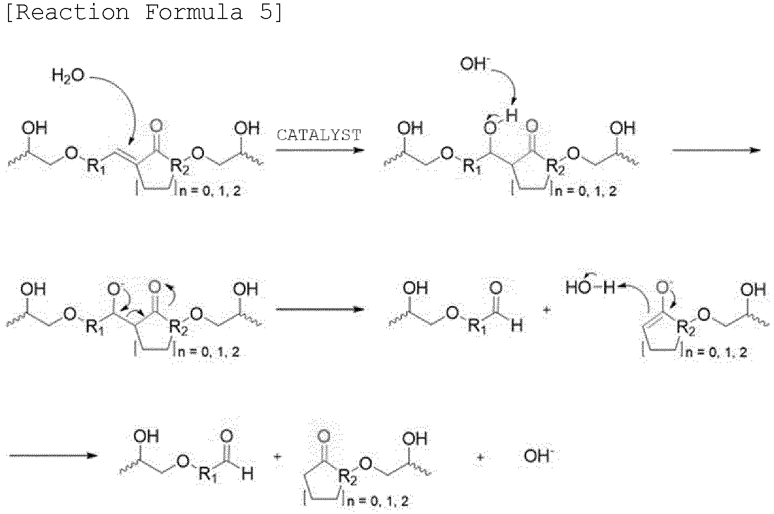

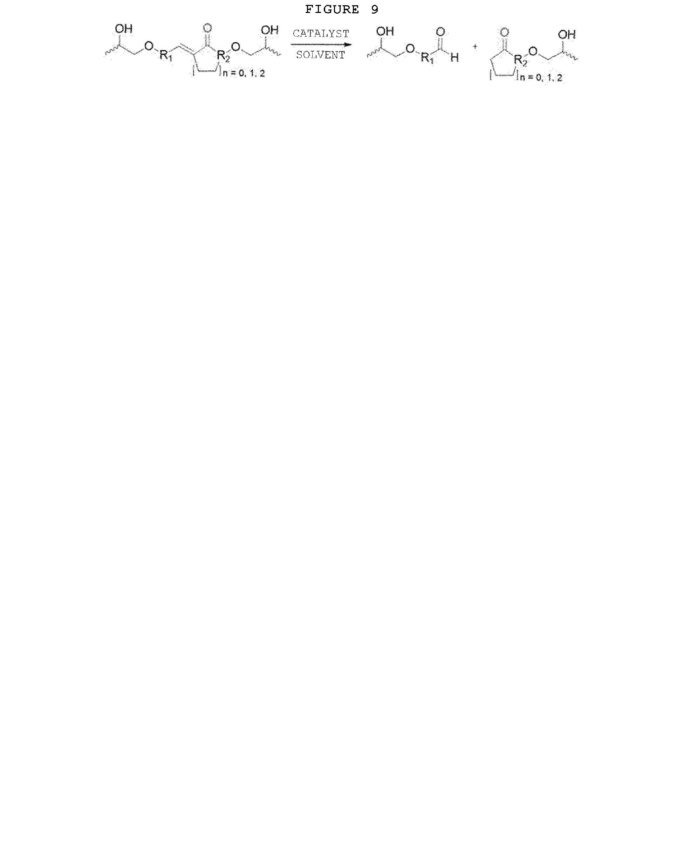

As illustrated in Reaction Formula 5, water molecules undergo an addition reaction in an unsaturated alkene to generate a ketone structure containing a hydroxyl group. Then, oxonium ions generated by a catalyst are added to the beta carbon in an unsaturated ketone to degrade the same into an aldehyde and a ketone molecule. Due to the electrons of the remaining ketone molecules, water is converted into oxonium ions, which are finally degraded into the two materials described above. Thus, the epoxy composite material is confirmed to be degradable. The results thereof can be confirmed by FIG. 9.

A sixth embodiment of the present disclosure provides a wind blade including the epoxy compound as raw material.

Glass fiber-reinforced plastics (GRP) are mainly used as materials for the wind blade. Even though iron, aluminum, and the like are also used, in the case of composite material blades, glass fibers and epoxy resins are the mainstream. As wind turbines are becoming gradually lightened and required to have excellent aging properties and high strength, the materials of the blade are being converted to epoxy resin composite materials.

On the other hand, the wind blade of the present disclosure uses the epoxy compound represented by Formula 3 and/or Formula 4, according to the first embodiment, as the raw material. As a result, the wind blade is chemically degradable and recyclable, so there is an environmentally-friendly advantage.

Hereinafter, the present disclosure will be described in more detail with reference to examples. However, the following examples are intended to illustrate the present disclosure, and the scope of the present disclosure is not limited thereto.

### <Example 1> Preparation of α,β-unsaturated ketone containing hydroxyl group through aldol reaction

4-hydroxy-3-methoxybenzaldehyde serving as a hydroxyl group-containing aldehyde compound of Formula 5 and 4-hydroxybenzylacetone serving as a ketone compound of Formula 6 were added to a flask in the same mole number.

250 mL of dichloromethane serving as a solvent and 25 mol% of N-ethylpiperidine serving as a catalyst were added to the flask to which the 4-hydroxy-3-methoxybenzaldehyde and 4-hydroxyphenylacetone were added, and then underwent a reaction at a temperature of 20°C for 40 hours.

The solvent was used for extraction after the reaction, and the extracted material was separated and purified using column chromatography. The material being separated and purified was subjected to heat treatment at a temperature of 75°C to prepare an α,β-unsaturated ketone containing a hydroxyl group.

### <Example 2> Preparation of α,β-unsaturated ketone containing hydroxyl group through aldol reaction

An α,β-unsaturated ketone containing a hydroxyl group was prepared in the same manner as in Example 1, except that 1-(hydroxymethyl)-cyclohexanecarboxaldehyde was used as the hydroxyl group-containing aldehyde compound of Formula 5, and 2,4-diacetyl-5-hydroxy-5-methyl-3-phenylcyclohexanone was used as the ketone compound of Formula 6.

### <Example 3> Preparation of α,β-unsaturated ketone containing hydroxyl group through aldol reaction

An α,β-unsaturated ketone containing a hydroxyl group was prepared in the same manner as in Example 1, except that 1-(hydroxymethyl)-4-isopropylcyclohexanecarbaldehyde was used as the hydroxyl group-containing aldehyde compound of Formula 5, and 2,4-diacetyl-5-hydroxy-5-methyl-3-phenylcyclohexanone was used as the ketone compound of Formula 6.

### <Example 4> Preparation of α,β-unsaturated ketone containing hydroxyl group through aldol reaction

An α,β-unsaturated ketone containing a hydroxyl group was prepared in the same manner as in Example 1, except that 4-(3-hydroxy-4-oxo-4H-1-benzopyran-2-yl)benzaldehyde was used as the hydroxyl group-containing aldehyde compound of Formula 5, and 5-acetyl-2-furanmethanol was used as the ketone compound of Formula 6.

### <Example 5> Preparation of α,β-unsaturated ketone containing hydroxyl group through aldol reaction

An α,β-unsaturated ketone containing a hydroxyl group was prepared in the same manner as in Example 1, except that 3-hydroxypropionaldehyde was used as the hydroxyl group-containing aldehyde compound of Formula 5, and 6-hydroxy-2-hexanone was used as the ketone compound of Formula 6.

### <Example 6> Preparation of α,β-unsaturated ketone containing hydroxyl group through aldol reaction

4-hydroxy-3-methoxybenzaldehyde serving as a hydroxyl group-containing aldehyde compound of Formula 5 and 4-hydroxybenzylacetone serving as a ketone compound of Formula 6 were added to a flask in the same mole number.

300 mL of dichloromethane serving as a solvent and 30 mol% of proline serving as a catalyst were added to the flask to which the 4-hydroxy-3-methoxybenzaldehyde and 4-hydroxybenzylacetone were added, and then underwent a reaction at a temperature of 20°C for 40 hours.

The solvent was used for extraction after the reaction, and the extracted material was separated and purified using column chromatography to prepare an α,β-unsaturated ketone containing a hydroxyl group.

### <Example 7> Preparation of α,β-unsaturated ketone epoxy

After the α,β-unsaturated ketone prepared in Example 1 was added to a flask, epichlorohydrin in excess mole number was added for a reaction. After being cooled to room temperature, sodium hydroxide was used as a base in an ice bath, and the resulting mixture then underwent a reaction for 7 hours.

A solvent was used for extraction after the reaction, and the extracted material was separated and purified using column chromatography to prepare an α,β-unsaturated ketone epoxy compound.

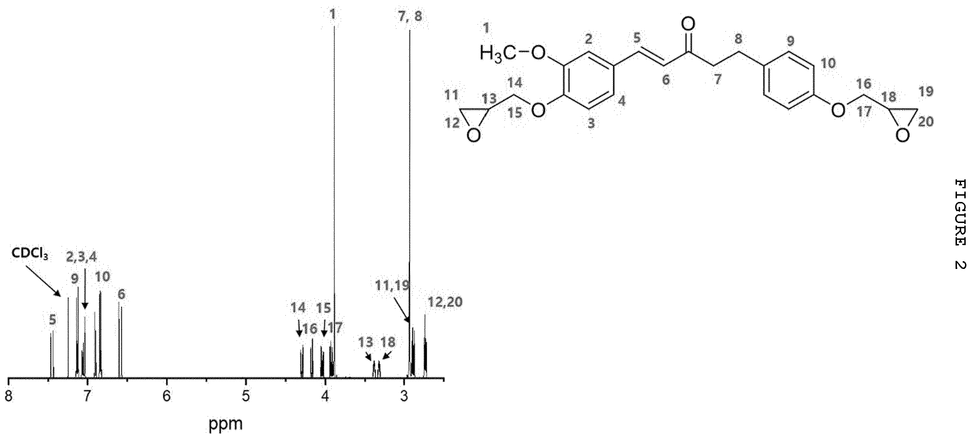

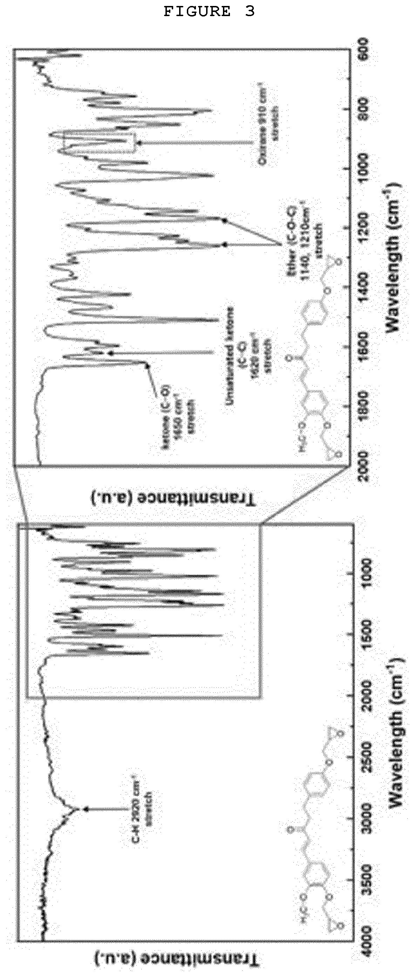

FIG. 2 is a diagram showing ¹H-NMR analysis results of the α,β-unsaturated ketone epoxy compound prepared above, and FIG. 3 is a diagram showing IR spectra of the α,β-unsaturated ketone epoxy compound prepared above.

### <Example 8> Preparation of α,β-unsaturated ketone epoxy

An α,β-unsaturated ketone epoxy compound was prepared in the same manner as in Example 7, except that the compound prepared in Example 2 was used as the α,β-unsaturated ketone.

### <Example 9> Preparation of α,β-unsaturated ketone epoxy

An α,β-unsaturated ketone epoxy compound was prepared in the same manner as in Example 7, except that the compound prepared in Example 3 was used as the α,β-unsaturated ketone.

### <Example 10> Preparation of α,β-unsaturated ketone epoxy

An α,β-unsaturated ketone epoxy compound was prepared in the same manner as in Example 7, except that the compound prepared in Example 4 was used as the α,β-unsaturated ketone.

### <Example 11> Preparation of α,β-unsaturated ketone epoxy

An α,β-unsaturated ketone epoxy compound was prepared in the same manner as in Example 7, except that the compound prepared in Example 5 was used as the α,β-unsaturated ketone.

### <Example 12> Preparation of α,β-unsaturated ketone epoxy

An α,β-unsaturated ketone epoxy compound was prepared in the same manner as in Example 7, except that the compound prepared in Example 6 was used as the α,β-unsaturated ketone.

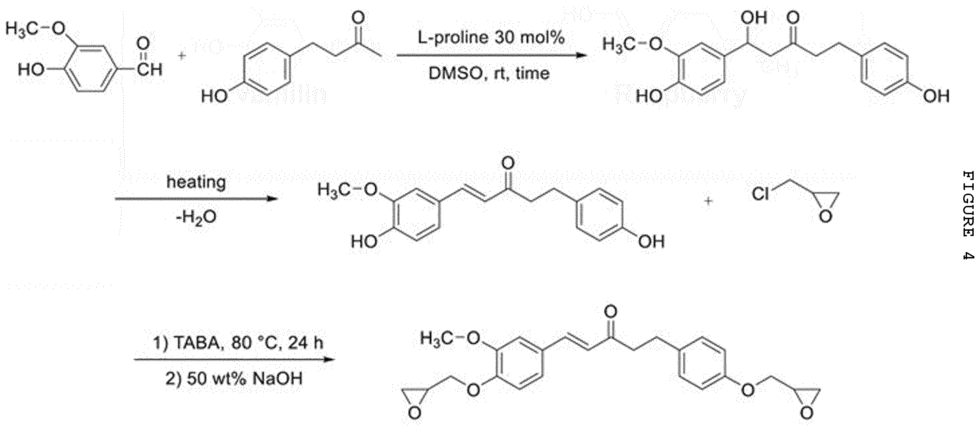

FIG. 4 is a diagram illustrating a reaction formula for the process of preparing the α,β-unsaturated ketone epoxy compound.

### <Example 13> Preparation of epoxy composite material containing α,β-unsaturated ketone epoxy and curing agent

The α,β-unsaturated ketone epoxy prepared in Example 7 and hexamethylenediamine serving as a curing agent were each independently added to a container, and then mixed using a mixer (2000 rpm, 10 minutes).

The well-mixed mixture was put into a mold and subjected to heat-pressure curing at a temperature of 200°C for 2 hours. The cured composite material containing the α,β-unsaturated ketone epoxy and the curing agent was polished to make the thickness thereof uniform.

### <Example 14> Degradation of epoxy composite material

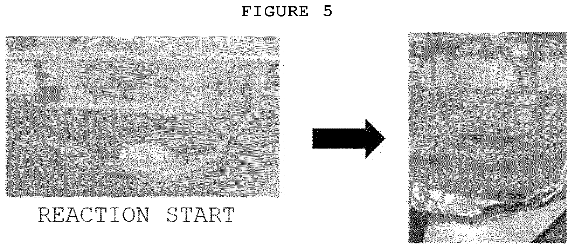

The epoxy composite material prepared in Example 13 was added to a flask and then underwent a reaction at a temperature of 20°C using titanium tetrachloride as a catalyst. The results thereof are shown in FIG. 5.

As shown in FIG. 5, it is confirmed that the cured epoxy composite material is completely degraded and thus recyclable.

### <Experimental Example 1>

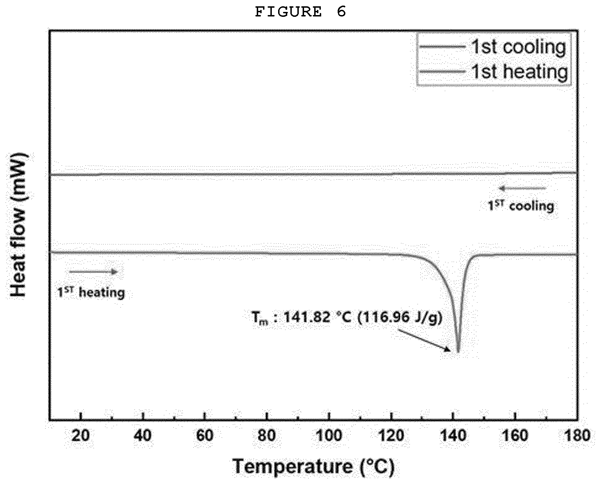

To examine the workability of the α,β-unsaturated ketone epoxy compound prepared in Example 7, a melting transition temperature was determined from differential scanning calorimetry (DSC) results. The results thereof are shown in FIG. 6.

### <Experimental Example 2>

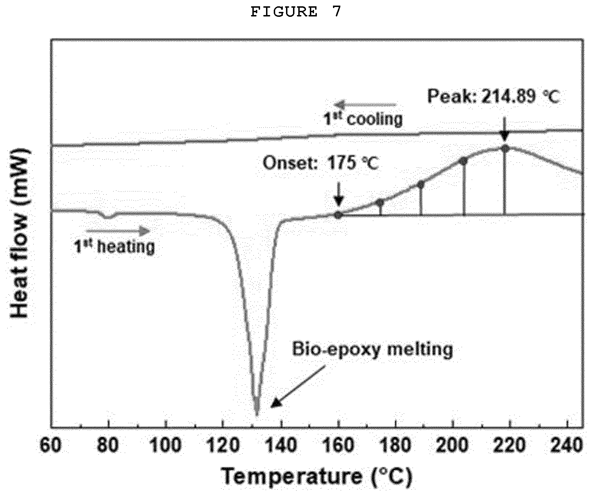

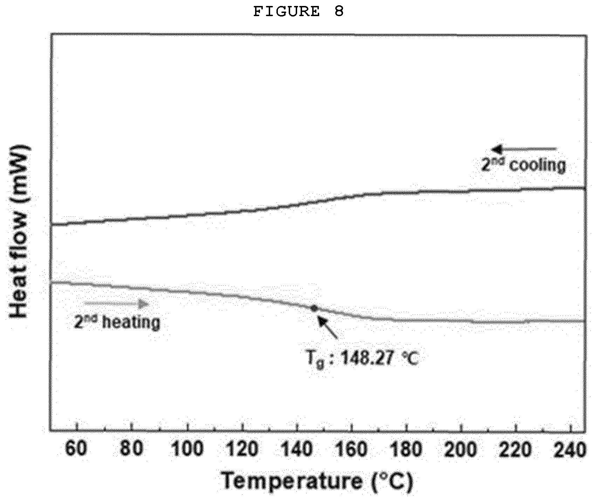

To examine the workability of the α,β-unsaturated ketone epoxy compound prepared in Example 7, a curing temperature and a glass transition temperature were determined from differential scanning calorimetry (DSC) results. The respective results thereof are shown in FIGS. 7 and 8.

As shown in FIG. 7, it is seen that the α,β-unsaturated ketone epoxy compound has a curing temperature in a range of 175°C to 245°C and a glass transition temperature in a range of 145°C to 155°C.

Hereinabove, particular features of the present disclosure are described in detail. However, it will be apparent to those skilled in the art that the specific description is merely a preferred embodiment, and the scope of the present disclosure is not limited thereto.

Thus, the substantial scope of the present disclosure will be defined by accompanying claims and equivalents thereof. It should be understood that simple modifications or changes can be made by those skilled in the art without departing from the spirit and scope of the disclosure.

### Industrial Applicability

The present disclosure can provide: a recyclable epoxy containing an α,β-unsaturated ketone group or hydroxy ketone group bonded through an aldol reaction between a ketone group and an aldehyde group containing a hydroxyl group among non-toxic natural materials, without using a bisphenol A type epoxy, a toxic material; a method of preparing the same epoxy; and a method of improving thermal properties, physical properties, and recyclability of an epoxy composite material by using the epoxy.

## Claims

1. A recyclable epoxy compound represented by Formula 3 or 4 and being chemically degradable, 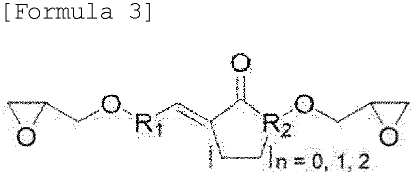 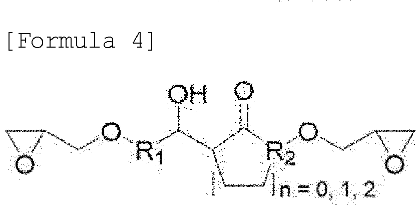 wherein in Formulas 3 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 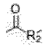
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

2. The compound of claim 1, wherein the compound represented by Formula 3 is a compound represented by Formula 7,

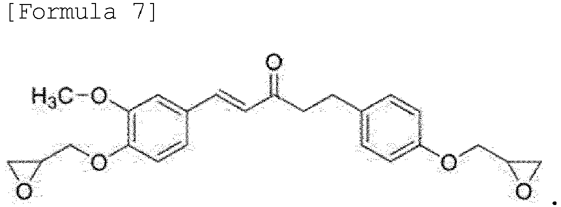

3. The compound of claim 1, wherein the compound represented by Formula 4 is a compound represented by Formula 8,

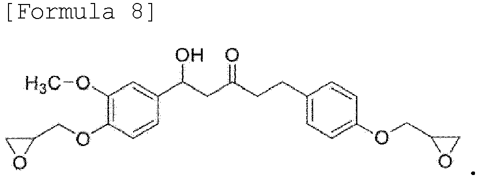

4. A method of preparing a recyclable epoxy compound represented by Formula 3 and being chemically degradable, the method comprising:
(a) causing an aldol reaction between an aldehyde group-containing compound and a ketone group-containing compound in the presence of a solvent and a catalyst to prepare a compound represented by Formula 1;
(b) performing heat treatment on the compound represented by Formula 1 to prepare a compound represented by Formula 2; and
(c) causing a reaction between the compound represented by Formula 2 and epichlorohydrin in the presence of a base, 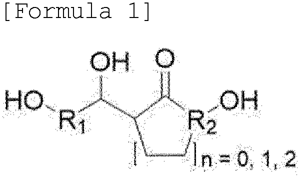 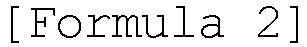 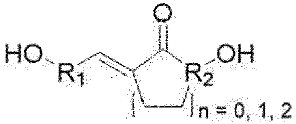 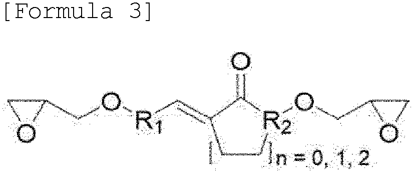 wherein in Formulas 1 to 3,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 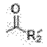
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

5. The method of claim 4, wherein the aldehyde group-containing compound is selected from the group consisting of 4-hydroxy-3-methoxybenzaldehyde, 4-hydroxybenzaldehyde, 3,4-dihydroxybenzaldehyde, p-hydroxycinnamaldehyde, 1-(hydroxymethyl)-cyclohexanecarboxaldehyde, 1-(hydroxymethyl)-4-isopropylcyclohexanecarbaldehyde, 3-hydroxycyclopentane-1-carbaldehyde, 4-hydroxycyclohexanecarboxaldehyde, 4-hydroxy-3-(2-propen-1-yl)cyclohexanecarboxaldehyde, 4-(hydroxymethyl)benzaldehyde, 2-(hydroxymethyl)benzaldehyde, 4-(2-hydroxyethyl)benzaldehyde, 5,6,9,10-tetrahydroxyphenanthro[9,10-b]furan-2-carboxaldehyde, 4-(3-hydroxy-4-oxo-4H-1-benzopyran-2-yl)benzaldehyde, trans-p-coumaraldehyde, (Z)-3-(4-hydroxy-3-methoxyphenyl)prop-2-enal, (E)-3-(4-hydroxy-3-methoxyphenyl)prop-2-enal, trans-4-(2,5dihydroxyphenyl)cyclohexanecarboxaldehyde, 4-hydroxy-7-benzofurancarboxaldehyde, 3-hydroxybutanal, 2,2-dimethyl-3hydroxypropane, glycolaldehyde, 3-hydroxypropionaldehyde, glyceraldehyde, 6-hydroxyhexanal, and hydroxypivalaldehyde.

6. The method of claim 4, wherein the ketone group-containing compound is selected from the group consisting of 4-(4-hydroxyphenyl)-2-butanone, 4'-hydroxyacetophenone, 4'-hydroxy-3'-methylacetophenone, 1-(6-hydroxy[1,1'-biphenyl]-3-yl)ethanone, 1-(4-hydroxycyclohexyl)ethanone, 4-hydroxybenzylacetone, 4-hydroxyphenylacetone, 2'-hydroxyacetophenone, 1-[4-[(1E)-2-hydroxyethenyl]phenyl]ethanone, 2,4-diacetyl-5-hydroxy-5-methyl-3-phenylcyclohexanone, (3E)-6-(4-hydroxyphenyl)-3-hexen-2-one, 2,4-diacetyl-5-hydroxy-3-(4-hydroxy-3-methoxyphenyl)-5-methylcyclohexanone, (3E)-4-(4-hydroxy-3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(4-hydroxyphenyl)-1-(3-methyl-1,2-benzisoxazol-5-yl)-2-propen-1-one, hydroxyacetone, 3-hydroxy-2-propanone, hydroxy-2-pentanone, 6-hydroxy-2-hexanone, 10-hydroxy-2-decanone, 8-hydroxy-2-octanone, 7-hydroxy-2-heptanone, (3S,4R)-3,4-dihydroxy-2,8-nonanedione, and 4-hydroxy-4-methyl-2,8-nonanedione.

7. The method of claim 4, wherein the solvent is selected from the group consisting of dichloromethane, tetrahydrofuran, hexamethylphosphoramide, hexane, toluene, dimethyl sulfoxide, ethanol, and water.

8. The method of claim 4, wherein the catalyst is selected from the group consisting of titanium tetrachloride, proline, N-ethylpiperidine, magnesium iodide, samarium trichloride, butyllithium, 1-piperazineethaneamine, diethylzinc, bis[(4R)-4-hydroxy-L-prolinato-κN1,κO2]copper, tin (II) triflate, boron trifluoride etherate, and tetraethylammonium p-toluenesulfonate.

9. The method of claim 4, wherein the solvent is water, and the catalyst is proline.

10. The method of claim 4, wherein the heat treatment is performed at a temperature in a range of 60°C to 90°C.

11. The method of claim 4, wherein the base is selected from the group consisting of sodium hydroxide, potassium carbonate, 4-methyl-2-phenyl-1H-imidazole-5-methanol, and hexadecyltrimethylammonium bromide.

12. The method of claim 4, wherein the reaction in the (c) is performed at a temperature in a range of 10°C to 50°C.

13. A method of preparing a recyclable epoxy compound represented by Formula 4 and being chemically degradable, the method comprising:
(d) causing an aldol reaction between an aldehyde group-containing compound and a ketone group-containing compound in the presence of a solvent and a catalyst to prepare a compound represented by Formula 1; and
(e) causing a reaction between the compound represented by Formula 1 and epichlorohydrin in the presence of a base, 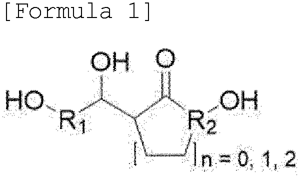 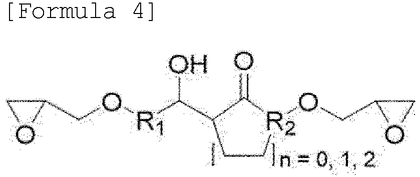 wherein in Formulas 1 and 4,
R₁ is selected from the group consisting of 3-methoxybenzene, benzene, hydroxybenzene, cinnamate, 1-methyl-cyclohexane, 1-methyl-4-isopropylcyclohexane, cyclopentane, cyclohexane, 3-(2-propen-1-yl)cyclohexane, 4-(methyl)benzene, 2-(methyl)benzene, 4-(2-ethyl)benzene, phenanthro[9,10-b]furan, 4-(4-oxo-4H-1-benzopyran-2-yl)benzene, trans-p-coumar, (Z)-3-(3-methoxyphenyl)prop-2-enal, (E)-3-(3-methoxyphenyl)prop-2-enal, trans-4-(hydroxyphenyl)cyclohexanecarboxaldehyde, 7-benzofuran, 3-butanal, 2,2-dimethyl-propane, glycol, propionate, 2-phenylethanol, 1,2-ethanediol, 1-phenyl-2,3-propanediol, 1-phenyl-2,3-butanediol, hexanal, and pivalate, and 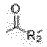
is selected from the group consisting of 4-(phenyl)-2-butanone, acetophenone, 3'-methylacetophenone, 1-([1,1'-biphenyl]-3-yl)ethanone, 1-(cyclohexyl)ethanone, benzylacetone, phenylacetone, 1-[4-[(1E)-2-ethenyl]phenyl]ethanone, 2,4-diacetyl-5-methyl-3-phenylcyclohexanone, (3E)-6-(phenyl)-3-hexen-2-one, 2,4-diacetyl-5-hydroxy-3-(3-methoxyphenyl)-5-methylcyclohexanone, (3E)-4-(3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(phenyl)-1-(3-methyl-1,2-benzisoxazol-5-yl)-2-propen-1-one, acetone, 2-propanone, 2-pentanone, 2-hexanone, 2-decanone, 2-octanone, 2-heptanone, (3S,4R)-2,8-nonanedione, and 4-methyl-2,8-nonanedione.

14. The method of claim 13, wherein the aldehyde group-containing compound is selected from the group consisting of 4-hydroxy-3-methoxybenzaldehyde, 4-hydroxybenzaldehyde, 3,4-dihydroxybenzaldehyde, p-hydroxycinnamaldehyde, 1-(hydroxymethyl)-cyclohexanecarboxaldehyde, 1-(hydroxymethyl)-4-isopropylcyclohexanecarbaldehyde, 3-hydroxycyclopentane-1-carbaldehyde, 4-hydroxycyclohexanecarboxaldehyde, 4-hydroxy-3-(2-propen-1-yl)cyclohexanecarboxaldehyde, 4-(hydroxymethyl)benzaldehyde, 2-(hydroxymethyl)benzaldehyde, 4-(2-hydroxyethyl)benzaldehyde, 5,6,9,10-tetrahydroxyphenanthro[9,10-b]furan-2-carboxaldehyde, 4-(3-hydroxy-4-oxo-4H-1-benzopyran-2-yl)benzaldehyde, trans-p-coumaraldehyde, (Z)-3-(4-hydroxy-3-methoxyphenyl)prop-2-enal, (E)-3-(4-hydroxy-3-methoxyphenyl)prop-2-enal, trans-4-(2,5dihydroxyphenyl)cyclohexanecarboxaldehyde, 4-hydroxy-7-benzofurancarboxaldehyde, 3-hydroxybutanal, 2,2-dimethyl-3hydroxypropane, glycolaldehyde, 3-hydroxypropionaldehyde, glyceraldehyde, 6-hydroxyhexanal, and hydroxypivalaldehyde.

15. The method of claim 13, wherein the ketone group-containing compound is selected from the group consisting of 4-(4-hydroxyphenyl)-2-butanone, 4'-hydroxyacetophenone, 4'-hydroxy-3'-methylacetophenone, 1-(6-hydroxy[1,1'-biphenyl]-3-yl)ethanone, 1-(4-hydroxycyclohexyl)ethanone, 4-hydroxybenzylacetone, 4-hydroxyphenylacetone, 2'-hydroxyacetophenone, 1-[4-[(1E)-2-hydroxyethenyl]phenyl]ethanone, 2,4-diacetyl-5-hydroxy-5-methyl-3-phenylcyclohexanone, (3E)-6-(4-hydroxyphenyl)-3-hexen-2-one, 2,4-diacetyl-5-hydroxy-3-(4-hydroxy-3-methoxyphenyl)-5-methylcyclohexanone, (3E)-4-(4-hydroxy-3-methoxyphenyl)3-buten-2-one, 4,7-anhydro-1,3-dideoxy-D-allo-2-octulose, 4,8-anhydro-1,3-dideoxy-D-allo-2-octulose, 5-acetyl-2-furanmethanol, 3-(4-hydroxyphenyl)-1-(3-methyl-1,2-benzisoxazol-5-yl)-2-propen-1-one, hydroxyacetone, 3-hydroxy-2-propanone, hydroxy-2-pentanone, 6-hydroxy-2-hexanone, 10-hydroxy-2-decanone, 8-hydroxy-2-octanone, 7-hydroxy-2-heptanone, (3S,4R)-3,4-dihydroxy-2,8-nonanedione, and 4-hydroxy-4-methyl-2,8-nonanedione.

16. The method of claim 13, wherein the solvent is selected from the group consisting of dichloromethane, tetrahydrofuran, hexamethylphosphoramide, hexane, toluene, dimethyl sulfoxide, ethanol, and water.

17. The method of claim 13, wherein the catalyst is selected from the group consisting of titanium tetrachloride, proline, N-ethylpiperidine, magnesium iodide, samarium trichloride, butyllithium, 1-piperazineethaneamine, diethylzinc, bis[(4R)-4-hydroxy-L-prolinato-κN1,κO2]copper, tin (II) triflate, boron trifluoride etherate, and tetraethylammonium p-toluenesulfonate.

18. The method of claim 13, wherein the solvent is water, and the catalyst is proline.

19. The method of claim 13, wherein the base is selected from the group consisting of sodium hydroxide, potassium carbonate, 4-methyl-2-phenyl-1H-imidazole-5-methanol, and hexadecyltrimethylammonium bromide.

20. The method of claim 13, wherein the reaction in the (e) is performed at a temperature in a range of 10°C to 50°C.

21. An epoxy composite material comprising:
at least one epoxy compound selected from the group consisting of the epoxy compounds represented by Formulas 3 and 4 of claim 1; and
a curing agent.

22. The material of claim 21, wherein the curing agent is selected from the group consisting of 2,6-dimethylaniline, hexamethylenediamine, isophorone diamine, diaminodiphenyl sulfone, 1,3benzenedimethaneanine, and dicyandiamide.

23. A method of degrading an epoxy composite material, the method comprising causing a reaction of the epoxy composite material of claim 21 or 22 in the presence of a catalyst.

24. The method of claim 23, wherein the catalyst is selected from the group consisting of titanium tetrachloride, ammonium chloride, trifluoroacetic acid, hydroxyamine hydrochloride, potassium hydroxide, sodium isopropoxide, tetrabutylammonium hydroxide, ethylene glycol, pyrrolidine, sodium periodate, cesium carbonate, alumina, and silver.

25. The method of claim 23, wherein the reaction is performed at a temperature in a range of 0°C to 40°C.

26. A wind blade comprising the epoxy compound of any one of claims 1 to 3 as a raw material.
